## Europäisches Patentamt
## European Patent Office
## Office européen des brevets

(19)

(11) Numéro de publication: **0 401 078**
**A1**

(12) **DEMANDE DE BREVET EUROPEEN**

(21) Numéro de dépôt: **90401306.7**

(22) Date de dépôt: **16.05.90**

(51) Int. Cl.⁵: **A61B 5/12**

La demande, qui etait incomplète au moment du dépot, est publiée telle quelle (article 93 (2) CBE). Le passage de la description ou des revendications qui comporte manifestement une omission est présenté comme tel.

Une requête en rectification concernant la revendication no.1 a été présentée conformément à la règle 88 CBE. Il est statué sur cette requête au cours de la procédure engagée devant la division d'examen (Directives relatives à l'examen pratiqué à l'OEB, A-V, 2.2).

(30) Priorité: **31.05.89 FR 8907491**

(43) Date de publication de la demande:
**05.12.90 Bulletin 90/49**

(84) Etats contractants désignés:
**AT BE CH DE ES FR GB GR IT LI LU NL SE**

(71) Demandeur: **Bourdin, Dominique**
**30 rue du Moulin, Vers sur Selle**
**F-80480 Saleux(FR)**

Demandeur: **Fabre, Dominique**
**201 rue J.Martinache, Auchy les Orchies**
**F-59310 Orchies(FR)**

Demandeur: **Vanloot, Daniel**
**320 Boulevard du 8 mai**
**F-62100 Calais(FR)**

(72) Inventeur: **Bourdin, Dominique**
**30 rue du Moulin, Vers sur Selle**
**F-80480 Saleux(FR)**
Inventeur: **Fabre, Dominique**
**201 rue J.Martinache, Auchy les Orchies**
**F-59310 Orchies(FR)**
Inventeur: **Vanloot, Daniel**
**320 Boulevard du 8 mai**
**F-62100 Calais(FR)**

(74) Mandataire: **Ecrepont, Robert**
**CABINET ECREPONT ROBERT 12, Place**
**Simon Vollant**
**F-59800 Lille(FR)**

(54) **Dispositif de traitement par voie auditive.**

(57) L'invention se rapporte à un dispositif de traitement par voie auditive comprenant notamment un capteur des sons générés par une source (3) et les adressant à un circuit (4) d'analyse en vue de déterminer la fréquence de chacun d'eux et notamment la tranche de fréquences à laquelle il appartient, un circuit de traitement (5) de chaque son en fonction de la tranche de fréquences à laquelle il appartient, lequel traitement s'opère sous le contrôle d'un moyen de commande (6) qui a au préalable été programmé manuellement ou automatiquement par un moyen à cet effet (14) en fonction des résultats d'un examen conduit à l'aide d'un audiomètre (7) sur au moins un patient, le signal ainsi traité (8) est envoyé à des moyens (9) de transmission à l'oreille.

Il est caractérisé en ce qu'il comprend des moyens (15) qui, alternativement et de façon aléatoire dans le temps, filtrent au moins un domaine prédéterminé de la partie de la bande des fréquences audibles.

Application à l'industrie du matériel médical.

## DISPOSITIF DE TRAITEMENT PAR VOIE AUDITIVE

L'invention se rapporte à un dispositif de traitement par voie auditive.

A ce jour, il est admis que l'oreille interne et l'aire corticale correspondante sont un hologramme de l'homme.

Chaque partie du corps a en effet une correspondance dans les fréquences sonores comme lumineuses.

Tout problème d'ordre psychosomatique laisse une trace dans ce système physiologique, trace repèrable à des modifications de la façon d'entendre sous les fréquences correspondantes.

Le repérage d'une fréquence auditive perturbée permet le diagnostic d'un problème d'ordre psychosomatique avant même que la lésion organique ne soit constituée.

Le diagnostic en permet le traitement à la fois préventif et curatif.

Afin d'objectiver les modifications de la façon d'entendre, on connaît bien entendu les audiométres, lesquels comprennent (FR-A-2.562.786) un générateur de sons apte à produire des sons de fréquences et d'intensités différentes, un casque d'écoute, un commutateur commandé en réponse aux sons perçus et des moyens d'analyse des réponses.

Avec ces audiomètres, les tests qui peuvent être conduits permettent de connaître la façon d'entendre sous les fréquences qui ont été testées.

Généralement, pour disposer de résultats s'échelonnant sur toute la bande passante, celle-ci est découpée en tranches de fréquences dans chacune desquelles est réalisé au moins un des tests précités, lesquels sont souvent conduits dans un ordre aléatoire.

Généralement, ces résultats sont utilisés pour automatiquement ou manuellement programmer une consigne d'un appareil de correction auditive communément dit prothèse auditive, qui sera intercalé entre une source sonore et l'oreille de manière quasi constante par l'usager et qui en fonction des résultats précités modulera en fonction de la fréquence des sons reçus l'intensité de la transmission à l'oreille mais respectera l'intégrité du message reçu et la source.

Le dispositif objet de la présente invention a une toute autre vocation.

Il est destiné à être porté pendant une durée très limitée et n'a pas à respecter l'intégralité du message sonore fourni par une source.

Comme la prothèse, il comprend un capteur de sons générés par une source, un circuit d'analyse des sons captés pour identifier, en référence au tranchage de la bande de fréquence réalisé dans l'audiomètre, la tranche de fréquences à laquelle ils appartiennent, une consigne de modulation des sons en fonction de la tranche de fréquences à laquelle ils appartiennent, un circuit de traitement des sons générés sous le contrôle de la consigne et un moyen de transmission des signaux issus du traitement.

Etant donnée sa différence de vocation, on comprend que le circuit de traitement et la consigne sont très différents de ceux d'une prothèse auditive.

Un des résultats que l'invention vise à obtenir est un dispositif de traitement par voie auditive permettant un traitement audiopsychophonologique.

Un des autres résultats de l'invention est un tel dispositif qui soit efficace et simple à utiliser.

A cet effet, elle a notamment pour objet un tel dispositif notamment caractérisé en ce qu'il comprend des moyens qui, alternativement et de façon aléatoire dans le temps, filtrent au moins un domaine prédéterminé de la bande des fréquences audibles.

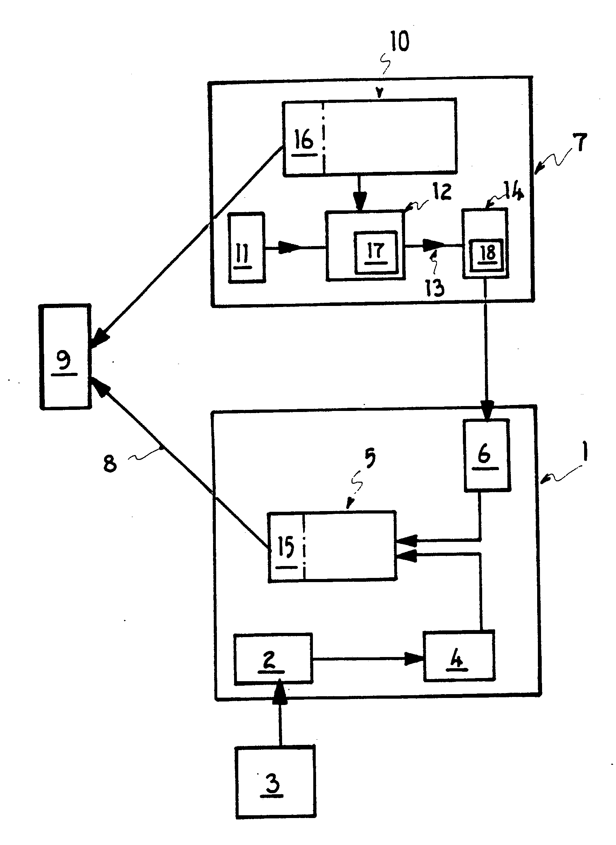

En se reportant au dessin, on voit que le dispositif de traitement 1 comprend de manière connue un capteur 2 des sons générés par une source 3 et les adressant à un circuit 4 d'analyse en vue de déterminer la fréquence de chacun d'eux et notamment la tranche de fréquences à laquelle il appartient, un circuit de traitement 5 de chaque son en fonction de la tranche de fréquences à laquelle il appartient, lequel traitement s'opère sous le contrôle d'un moyen de commande 6 qui a, au préalable, été programmé manuellement ou automatiquement, par un moyen 14 à cet effet en fonction des résultats d'un examen conduit à l'aide d'un audiomètre 7 sur au moins un patient (non représenté), le signal ainsi traité 8 étant envoyé à des moyens 9 de transmission à l'oreille.

Classiquement, l'audiomètre 7 comprend un générateur de sons 10, apte à produire des sons de fréquences et d'intensités différentes, au moins un casque d'écoute 9, au moins un commutateur 11 commandé par au moins un patient ou un tiers en réponse aux sons perçus et des moyens 12 d'analyse des réponses.

Ce sont ces résultats 13 de l'analyse qui sont alors manuellement ou via un moyen de programmation 14 utilisés pour programmer le moyen de commande 6 du dispositif de traitement 1.

Selon une caractéristique essentielle de l'invention, le dispositif de traitement comprend des moyens 15 qui, alternativement et de façon aléatoire dans le temps, filtrent au moins un domaine prédéterminé de la bande des fréquences audibles.

Suivant l'invention, au moins l'un des moyens de commande 6 et/ou de programmation 14 détermine de manière aléatoire la position et/ou la largeur de chaque domaine précité filtré dans la bande de fréquences audibles.

Les moyens 15 de filtration opèrent une filtration au moins partielle.

Il s'agit de filtres en fréquence ou en intensité.

Le moyen de transmission 9 est de préférence un casque et de plus il s'agit, de préférence, du même casque que celui ayant servi pour l'audiométrie préalable.

Indépendamment de cette filtration qui, de manière aléatoire dans le temps, élimine sensiblement alternativement les aigus et les graves, les circuits de traitement 5 en combinaison avec le moyen de commande 6 modulent en puissance les sons en fonction de leur tranche de fréquences pour au moins baisser sinon supprimer les sons dans les fréquences où une hypersensibilité a été détectée et pour au contraire monter les sons dans les fréquences où une hyposensibilité a été détectée.

La source 3 est par exemple un magnétophone, une platine, etc ...

L'audiomètre 7 est lui-même caractérisé en ce qu'il comprend un moyen 16 modulant en fréquence autour de chaque point de mesure de l'audiomètre, les sons purs émis par le générateur de sons 10 de manière que l'ensemble des stimuli auditifs couvrent toute la bande des fréquences audibles.

Les moyens de modulation s'opèrent selon une séquence aléatoire ou progressive.

Selon l'invention, le dispositif de traitement comprend, d'une part, au moins un module 17 de détection de points dits singuliers c'est à dire de points de mesure qui, suite à au moins une première audiomètrie, révèlent une hypersensibilité ou une hyposensibilité d'un patient dans au moins un domaine particulier de fréquences et, d'autre part, au moins un module 18 de commande du générateur 10 de sons en vue d'établir avec le patient concerné au moins une seconde audiométrie de part et d'autre de chaque point singulier avec une précision accrue par rapport à la dite première audiométrie.

De préférence, mais non limitativement pour l'invention, le module 17 de détection est interne au moyen 12 d'analyse tandis que le module 18 de commande est interne au moyen 14 de programmation.

Les applications de ce dispositif de traitement par élimination aternative des aigus et des graves de manière aléatoire dans le temps et donc sans référence par exemple à la puissance
sont nombreuses :
- dépistage de troubles psychosomatiques avant la constitution de la lésion,
- dépistage de l'origine ou de la composante psychologique de troubles somatiques,
- dépistage de l'origine de troubles dyslexiques ou de difficultés d'apprentissage chez l'enfant,
- correction des troubles dépistés d'où :
chez les enfants :
. amélioration de l'apprentissage,
. amélioration de leur comportement,
. facilitation de leur insertion sociale et professionnelle,
. amélioration de leur place dans la société,
. diminution des laissés pour compte,
chez les adultes :
. être mieux dans sa peau, donc limitation des risques de tomber malade, de somatiser des problèmes psychiques,
. amélioration de la rentabilité professionnelle,
. amélioration de l'insertion sociale,
. amélioration de la capacité d'apprentissage, d'où facilitation à la reconversion.

Outre la correction des troubles dépistés, le seul fait de stimuler le tympan de la façon proposée entraîne une stimulation du cerveau et améliore considérablement les facultés d'apprentissage probablement en multipliant les connexions neuronales d'où d'autres applications telles que :
- traitement des maladies essentiellement psychosomatiques dans leur composant psychique (allergies, dépression, dyslexies, angoisses, spasmophylie, troubles du sommeil ...),
- aide au traitement des maladies somatiques par correction de leur composante psychique.

De préférence, le dispositif selon l'invention est conçu pour son utilisation simultanée avec plusieurs patients.

## Revendications

2. Dispositif selon la revendication 1 **caractérisé** en ce qu'au moins l'un des moyens de commande (6) et/ou de programmation (14) détermine de manière aléatoire la position et/ou la largeur de chaque domaine précité filtré dans la bande de fréquences audibles.

3. Dispositif selon la revendication 1 **caractérisé** en ce les moyens (15) de filtration opèrent une filtration au moins partielle.

4. Dispositif selon la revendication 1 **caractérisé** en ce que les moyens (15) de filtrations sont des filtres en fréquence.

5. Dispositif selon la revendication 1 **caractérisé** en ce que les moyens (15) de filtration sont des filtres en intensité.

6. Dispositif selon la revendication 1 comprenant en outre un audiomètre (7) comprenant un générateur de sons (10), apte à produire des sons de fréquences et d'intensités différentes, au moins un casque d'écoute (9), au moins un commutateur

(11 commandé par au moins un patient ou un tiers en réponse aux sons perçus et des moyens (12) d'analyse des réponses, ces résultats (13) de l'analyse étant alors manuellement ou via un moyen de programmation (14) utilisés pour programmer le moyen de commande (6) du dispositif de traitement (1),

ce dispositif étant **caractérisé** en ce que le dit audiomètre comprend un moyen (16) modulant en fréquence autour de chaque point de mesure de l'audiométrie, les sons purs émis par le générateur de sons (10) de manière que l'ensemble des stimuli auditifs couvrent toute la bande des fréquences audibles.

7. Dispositif selon la revendication 6 **caractérisé** en ce que les moyens de modulation s'opèrent selon une séquence aléatoire.

8. Dispositif selon la revendication 6 **caractérisé** en ce que les moyens de modulation s'opèrent selon une séquence progressive.

9. Dispositif selon la revendication 6 **caractérisé** en ce qu'il comprend, d'une part, au moins un module (17) de détection de points dits singuliers c'est à dire de points de mesure qui, suite à au moins une première audiométrie, révèlent une hypersensibilité ou une hyposensibilité d'un patient dans au moins un domaine particulier de fréquences et, d'autre part, au moins un module (18) de commande du générateur (10) de sons en vue d'établir avec le patient concerné au moins une seconde audiométrie de part et d'autre de chaque point singulier avec une précision accrue par rapport à la dite première audiométrie.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,A | FR-A-2562786 (INTECH SYSTEMS CORP.) <br> * page 3, ligne 13 - page 8, ligne 2; figures 1, 2 * | 1, 6 | A61B5/12 |
| A | US-A-4191175 (NAGLE) <br> * colonne 1, lignes 7 - 48 * <br> * colonne 2, ligne 19 - colonne 3, ligne 8 * <br> * colonne 4, ligne 42 - colonne 5, ligne 49 * <br> * colonne 6, lignes 43 - 59; figure 1 * | 1-4 | |
| A | US-A-4107465 (CHARLEBOIS ET AL) <br> * colonne 4, ligne 4 - colonne 7, ligne 37; figures 1a-3 * | 1-3, 5-8 | |
| A | WO-A-8500509 (WESTRA ELECTRONIC GMBH) <br> * abrégé * <br> * page 10, ligne 22 - page 12, ligne 20 * <br> * page 14, ligne 21 - page 21, ligne 21 * <br> * page 23, ligne 26 - page 25, ligne 25 * <br> * page 28, ligne 27 - page 30, ligne 23; figures 1-5, 16 * | 1 | DOMAINES TECHNIQUES RECHERCHES (Int. Cl.5) <br><br> A61B |

Le présent rapport a été établi pour toutes les revendications

| Lieu de la recherche | Date d'achèvement de la recherche | Examinateur |
|---|---|---|
| LA HAYE | 07 SEPTEMBRE 1990 | CHEN A.H. |